# EUROPÄISCHE PATENTSCHRIFT

(11) **EP 1 624 827 B1**
(45) Veröffentlichungstag und Bekanntmachung des Hinweises auf die Patenterteilung: **28.11.2012**
(21) Anmeldenummer: 04727541.7
(22) Anmeldetag: 15.04.2004
(51) Int. Cl.: A61F 2/90, A61L 31/14

(54) **STENTS AUS EINEM MATERIAL GERINGER BRUCHDEHNUNG**
STENTS MADE OF A MATERIAL HAVING A LOW ELONGATION AT FRACTURE
STENTS CONSTITUES D'UN MATERIAU PRESENTANT UN ALLONGEMENT A LA RUPTURE REDUIT

(30) Priorität: 20.05.2003 DE 10323628
(43) Veröffentlichungstag der Anmeldung: 15.02.2006
(73) Patentinhaber: Biotronik AG, 8180 Bülach (CH)
(72) Erfinder: LOOTZ, Daniel, 18119 Rostock-Warnemünde (DE); KOOP, Karsten, 18057 Rostock (DE); BECHER, Bärbel, 18057 Rostock (DE); KIEKBUSCH, Martin, 18437 Stralsund (DE); BAKCZEWITZ, Frank, 18055 Rostock (DE)
(74) Vertreter: Lindner-Vogt, Karin L.
(86) Internationale Anmeldenummer: PCT/EP2004/003999
(87) Internationale Veröffentlichungsnummer: WO 2004/103215

(56) Entgegenhaltungen:
- EP-A- 1 066 804
- WO-A-01/26584
- DE-A- 19 731 021
- US-A- 5 922 020
- US-A1- 2002 013 619

## Beschreibung

Die Erfindung betrifft Stents aus einem Material mit einer Bruchdehnung von 30% oder weniger und mit einem rohrförmigen Grundkörper, der ganz oder in Teilen aus Tragsegmenten besteht, die in Längsrichtung der Stents über Querverbinder miteinander verbunden sind, und bei denen die Tragsegmente eine zickzack- oder wellenförmige Struktur aus einer um eine Längsachse der Stents umlaufenden Strebe umfassen.

Koronare Herzerkrankungen, insbesondere akute Myokardinfarkte, stellen in Westeuropa und Nordamerika eine der häufigsten Todesursachen dar. In mehr als 80% der Fälle ist die Ursache des Myokardinfarktes der thrombotische Verschluss einer Koronararterie durch Ruptur einer atheromatösen Plaque bei vorbestehender stenosierender Atheromatose. Entscheidende Faktoren für die Langzeitprognose nach akutem Myokardinfarkt sind:
- eine effektive und langanhaltende Wiedereröffnung der Infarktarterie,
- eine Dauer des thrombotischen Gefäßverschlusses,
- die Verhinderung eines größeren Myokardverlustes und eines ventrikulären Remodeling,
- die Beherrschung rhythmogener Komplikationen.

Die genannten Faktoren bestimmen nicht nur die kardiovaskuläre Mortalität, sondern auch die Lebensqualität nach dem Infarkt.

Seit mehr als zwanzig Jahren sind nicht-operative Methoden zur Stenose-Behandlung etabliert, bei denen u.a. durch Ballondilatation (PTCA Perkutane Transluminale Coronare Angioplastie) das verengte oder verschlossene Blutgefäß wieder aufgeweitet wird. Dieses Vorgehen hat sich insbesondere bei der Therapie des akuten Myokardinfarktes bewährt. Nach dem Aufweiten des Blutgefäßes entstehen in etwa einem Drittel der Fälle durch das durch die Behandlung ausgelöste Zellwachstum überdurchschnittliche Wucherungen, die letztendlich zu einer erneuten Gefäßverengung (Restenose) führen. Eine weitere Ursache der Restenose ist die Elastizität des gedehnten Blutgefäßes. Nach dem Entfernen des Ballons zieht sich das Blutgefäß übermäßig zusammen, so dass der Gefäßquerschnitt verringert wird (Obstruktion, sogenanntes negatives remodeling). Letzterer Effekt kann nur durch Platzierung eines Stents vermieden oder zumindest behindert werden.

In der interventionellen Therapie der stabilen Angina pectoris bei koronarer Herzkrankheit, hat die Einführung der Stents zu einer deutlichen Reduktion der Rate an Restenosen und damit zu besseren Langzeitresultaten geführt. Dies gilt sowohl für die primäre als auch die Rezidivstenose. Ursächlich für den Nutzen der Stent-Implantation ist der höhere primäre Lumengewinn.

Durch den Einsatz von Stents kann zwar ein optimaler Gefäßquerschnitt erreicht werden. Allerdings initiiert die Anwesenheit eines derartigen Fremdkörpers eine Kaskade von mikrobiologischen Prozessen, die zu einem allmählichen Zuwachsen des Stents führen können.

Der Stent besteht in der Regel aus einem rohrförmigen Grundkörper mit einer Umfangswandung aus Streben und Querverbindern zwischen den sich Zellen unterschiedlichster Formgestaltung erstrecken. Ein gemeinsames Merkmal aller gängigen Stents besteht darin, dass ein wie auch immer geartetes Design der Stents eine Expansion von einem geschlossenen (nicht-expandierten) Zustand in einem aufgeweiteten (expandierten) Zustand ermöglichen muss. Im nicht-expandierten Zustand wird der Stent in den Bereich der Läsion des Blutgefäßes geführt. Am Applikationsort expandiert die Struktur, entweder durch von außen aufgebrachte Kräfte (z.B. mittels eines im Innern des Stents angeordneten Ballons) oder indem der Stent selbstexpandierend ausgelegt ist (z.B. durch Verwendung eines Gedächtnismaterials).

Ein Stentdesign muss insbesondere folgenden Erfordernissen genügen:
- Im expandierten Zustand soll der Stent eine möglichst große Fläche der Gefäßwand gleichmäßig abstützen.
- Eine Expansion des Stents in radialer Richtung unterstützen, ohne eine Ausdehnung in axialer Richtung zuzulassen.
- Die einzelnen Streben und Querverbinder sollen im nicht-expandierten und expandierten Zustand sowie während des Übergangs vom nicht-expandierten zum expandierten Zustand möglichst in einer gemeinsamen radialen Umlaufebene angeordnet sein, d. h. ein Herausragen einzelner Strukturelemente muss zur Vermeidung von Gefäßverletzungen unterdrückt werden.
- Ein Versagen des Materials muss über die Dauer des Heilungs- und Implantationszeitraums vermieden werden.

Moderne Stentdesigns tragen bei Verwendung gängiger Stentmaterialien den genannten Erfordernissen Rechnung. Beispiele solcher Stentdesigns sind beispielsweise in den Patentanmeldungen EP 1 066 804 A2, US 2002/0013619 A oder WO 01/26584 A1 ersichtlich.

Anfänglich wurden Stents zumeist aus medizinischem Stahl, z.B. 316L, hergestellt. Im Laufe der Zeit zeigte sich allerdings, dass die eingesetzten Materialien zwar biokompatibel, aber über mittlere und lange Zeiträume teils eine Thrombosebildung und teils eine Adhäsion von Biomolekülen an ihrer Oberfläche förderten. Ein Ansatzpunkt zur Lösung dieser Problematik sind Stents aus einem biodegradierbaren Material. Unter Biodegradation werden hydrolytische, enzymatische und andere stoffwechselbedingte Abbauprozesse im lebendem Organismus verstanden, die zu einer allmählichen Auflösung zumindest großer Teile des Implantats führen. So wurden beispielsweise eine Vielzahl von Kunststoffmaterialien als Stentwerkstoff vorgeschlagen, die zwar eine hohe Biokompatibilität und ein gutes Degradationsverhalten zeigten, jedoch aufgrund ihrer mechanischen Eigenschaften allenfalls eingeschränkt für die medizinische Applikation nutzbar sind.

Zur Überwindung dieses Nachteils wird der Einsatz spezieller biodegradierbarer Metalllegierungen angestrebt, wie sie insbesondere in der DE 197 31 021 und DE 199 45 049 - deren Offenbarung hiermit vollumfänglich einbezogen wird - beschrieben werden. Die Metalllegierungen umfassen spezielle Eisen-, Wolfram- und Magnesiumlegierungen. Die favorisierten Materialien haben allerdings zumindest teilweise den Nachteil, dass sie eine Bruchdehnung von 30% oder weniger besitzen und alle gängige Stentdesigns damit ausgeschlossen sind. So zeigt der Werkstoff 316 L eine Bruchdehnung von 40 - 50%, bestimmt an einer rohrförmigen Zugprobe mit dem Durchmesser 1,6 mm und einer Wandstärke von 0,1 mm, bei einer Probenlänge von 60 mm und einer Messlänge von 22 mm. Eine typische biodegradierbare Magnesiumlegierung hat eine Bruchdehnung von 14%, bestimmt an einer rohrförmigen Zugprobe mit dem Durchmesser 1,6 mm und einer Wandstärke von 0,2 mm, bei einer Probenlänge von 50 mm und einer Messlänge von 30 mm.

Unter dem Begriff "Bruch" wird hier die durch Überbeanspruchung hervorgerufene Trennung der Bindungen zwischen den atomaren Bestandteilen eines Werkstoffs entlang einer sich über die gesamte Breite der Werkstoffprobe erstreckenden Bruchfläche verstanden. Im Augenblick des Bruchs übersteigen die im Werkstoff durch äußere oder auch innere Kräfte aufgebauten Spannungen die werkstoffspezifische Bruchspannung und werden dadurch größer als die in der Bruchfläche wirkenden Bindungskräfte. Die Bruchfestigkeit besagt, mit welcher Kraft eine Werkstoffprobe bis zum Bruch belastbar ist. Verformungslose oder verformungsarme Brüche lassen sich mit den genannten Metalllegierungen problemlos kompensieren. Dagegen sind Verformungsbrüche wie Biege - und Torsionsbrüche mit herkömmlichen Stentdesigns unvermeidbar, denn das Material hat nur eine ungenügende Bruchdehnung.

Unter "Bruchdehnung" wird die verbleibende Verlängerung nach einem Zugversuch bis zum Bruch, d. h. der Längenunterschied zwischen ursprünglicher Probenlänge und gebrochener Probe, bezeichnet.

Aufgabe der vorliegenden Erfindung ist es, ein Stentdesign zu schaffen, das für Materialien mit einer Bruchdehnung von kleiner 30% geeignet ist.

Diese Aufgabe wird durch den Stent mit den Merkmalen des Anspruchs 1 gelöst.

Der erfindungsgemäße Stent besteht aus einer biodegradierbaren Metalllegierung mit einer Bruchdehnung von 30% oder weniger und einem rohrförmigen Grundkörper der ganz oder in Teilen aus Tragsegmenten besteht. Die Tragsegmente sind in Längsrichtung über Querverbinder miteinander verbunden und umfassen jeweils eine zickzack - oder wellenförmige Struktur aus einer um eine Längsachse des Stents umlaufenden Strebe. Der Stent zeichnet sich dadurch aus, dass die Strebe in einem Wendebereich der zickzack- oder wellenförmigen Struktur einen geraden, in Umfangsrichtung ausgerichteten Biegeabschnitt aufweist. Die erfindungsgemäßen Biegeabschnitte sind derart in das Stentdesign integriert, dass sie bei einer Expansion des Stents von einem nicht-expandierten in einen expandierten Zustand mit einem Spannungs- als auch einem Biegemoment beaufschlagt werden. Die erfindungsgemäße Ausrichtung und Form der Biegeabschnitte bezieht sich nur auf den nicht-expandierten Zustand, d.h. nach der Expansion weisen die Biegeabschnitte in der Regel eine andere Ausrichtung und Form auf. Die gerade Ausrichtung führt bei der Expansion zur gleichmäßigen Spannungs- und Dehnungsverteilung. Nur annähernd gerade Biegeabschnitte sind diesbezüglich nicht optimal und es lässt sich nur eine ähnlich, annähernd gleichmäßige Spannungs- und Dehnungsverteilung erzielen. Bei Verformung der Struktur werden diese geraden Biegeabschnitte hauptsächlich durch ein Biegemoment beansprucht. Aufgrund des konstanten Hebelarms bzw. des konstanten Moments über den gesamten geraden Biegeabschnitt verteilt sich die Dehnung gleichmäßig auf diesem Biegeabschnitt. Lokale Spannungs - und Dehnungskonzentrationen werden weitgehend vermieden und durch diese Maßnahme wird der geringen Bruchdehnung (<30%) Rechnung getragen.

Die Schenkelabschnitte sind so kurz gehalten, dass sie sich in Umfangsrichtung bei der Expansion des Stents aufrichten. Dadurch wird ein gleichmäßiges Aufweiten des Stents geometrisch erzwungen. Das bedeutet, dass sich bei zunehmender Aufweitung die Länge des Hebelarms der Schlaufen stark verkürzt und somit wesentlich mehr Kraft nötig wird sie zu öffnen. Da die Reaktionskräfte über den Umfang für jedes Segment konstant sind, öffnen sich somit erst andere Zellen, deren Schenkelabschnitte weniger steil stehen. Die Verfestigung des Materials ist so niedrig, dass sie allein nicht ausreichend wäre, das gleichmäßige Öffnen der Struktur bei einem herkömmlichen Stentdesign zu gewährleisten. Weiterhin wird durch das Aufstellen der Schenkelabschnitte die aufgrund des niedrigen E-Moduls normalerweise hohe Recoil minimiert, da in dieser Position der Rückfederungswinkel des Stents nur einen kleinen Einfluss auf den Rückfederungsweg hat. Ein Kollapsdruck wird ebenfalls günstig beeinflusst, da die Torsionsbeanspruchung der Strebe mit deren Aufrichten deutlich abnimmt.

In einer vorteilhaften Weiterbildung des erfindungsgemäßen Stentdesigns sind im Wendebereich der zickzack- oder wellenförmigen Struktur Anschlagelemente angeordnet. Diese sind so ausgebildet, dass Verformungen der zickzack- oder wellenförmigen Struktur, die in Folge einer Expansion von einem nicht-expandiertem in einen expandierten Zustand des Stents auftreten, im Bereich der geraden Biegeabschnitte vermindert und im Bereich der Schenkel verstärkt werden. Dies wird insbesondere dadurch erreicht, dass die Anschlagelemente jeweils aus zwei Stegen bestehen, die mit einem Ende an der zickzack- oder wellenförmigen Struktur fixiert sind. Die freien Enden der beiden Stege sind aufeinander gerichtet und so angeordnet, dass sie sich während der Expansion des Stents aufeinander zu bewegen bis sie aneinander liegen. Von diesem Zeitpunkt an wird die Verformung in den geraden Abschnitten begrenzt und in die weiteren Verformungszonen des Stents verlagert. Diese Maßnahme führt ebenfalls zu einer optimierten Verteilung der Dehnung in der Struktur.

Nach einer weiteren bevorzugten Ausgestaltung des Stentdesigns beträgt ein Verhältnis der Länge eines gerade verlaufenden Biegeabschnitts der Strebe zu seiner Breite 1:2 bis 1:6, vorzugsweise 1:2 bis 1:3. Von der Breite und Länge der Biegeabschnitte ist deren Dicke, also deren Erstreckung in radialer Richtung, zu unterscheiden. Es hat sich gezeigt, dass die genannten Vorgaben besonders zuverlässig einen Bruch des Werkstoffs verhindern können.

Es ist ferner bevorzugt, wenn der Stent doppel-s-förmige Schenkelabschnitte aufweist. Mit dieser Formgebung können die zuvor geschilderten günstigen Eigenschaften auf das Recoil-Verhalten und den Kollapsdruck unterstützt werden. Weiterhin ist es vorteilhaft die Schenkelabschnitte mit Sicht auf die bei der Expansion auftretenden Spannungsverhältnisse zu optimieren. Dazu wird auf die DE 100 44 043 der Anmelderin verwiesen. Weiterhin zeigt das für die Stents verwendete Material vorzugsweise eine Bruchdehnung von 5-30%, insbesondere 10-30%, besonders bevorzugt 10-25%, besonders bevorzugt 10-20%, besonders bevorzugt 10-15%. In den sich verkleinernden Vorzugsbereichen hat sich die Verwendung des Stentdesigns gegenüber alternativen Konstruktionen als besonders geeignet erwiesen.

Ferner ist bevorzugt, wenn die Tragsegmente in Längsrichtung des Stents derart angeordnet sind, dass die zickzack- oder wellenförmigen Strukturen wenigstens zwei benachbarter Tragsegmente in Phase verlaufen. Hierdurch kann eine besonders gleichmäßige Tragstruktur im expandierten Zustand erreicht und eine Längenänderung minimiert werden.

Vorzugsweise ist ferner die biodegradierbare Metalllegierung eine Wolfram-, Magnesium- oder Eisenlegierung.

Vorteilhaft ist weiterhin, dass die Querverbinder parallel zur Längsrichtung des Stents ausgerichtet sind. Eine solche Anordnung unterstützt die gleichförmige Ausdehnung der Struktur während der Expansion. Zur Erhöhung der Biegeflexibilität ist es weiterhin vorteilhaft, wenn die parallel zur Längsachse des Stents verlaufenden Querverbinder S-, V-, W- oder eine andere multi-sinus-förmige Formgebung aufweisen.

Prinzipiell ist ein Ansatzpunkt der Querverbinder frei entlang der umlaufenden zickzack - oder wellenförmigen Struktur wählbar. Es hat sich jedoch als vorteilhaft erwiesen, wenn ein Ansatzpunkt der Querverbinder im Bereich der geraden Biegeabschnitte, insbesondere in deren Mitte, liegt. Auch durch diese Maßnahme wird das Expansionsverhalten verbessert und eine gleichmäßigere Tragstruktur erzielt.

Die Ausrichtung der erfindungsgemäßen geraden Biegeabschnitte hat in Umfangrichtung des Stents zu erfolgen, wobei leichte Abweichungen aus der Vorgaberichtung ohne eine wesentliche Verschlechterung im Expansionsverhalten tolerierbar sind. Die Abweichung liegt vorzugsweise bei < 25°, insbesondere < 20°, besonders bevorzugt < 10°. Mit anderen Worten, eine Abweichung der Ausrichtung der geraden Biegeabschnitte in Umfangrichtung des Stents beträgt höchstens 25°, insbesondere höchstens 20°, besonders bevorzugt höchstens 10°.

Die Erfindung wird nachfolgend in Ausführungsbeispielen und anhand der dazugehörigen Zeichnungen näher erläutert. Es zeigen:
- Fig. 1: eine schematisierte Ausschnittsansicht auf ein erfindungsgemäßes Stentdesign nach einer ersten Variante,
- Fig. 2: eine schematisierte Ausschnittsansicht auf ein erfindungsgemäßes Stentdesign nach einer zweiten Variante,
- Fig. 3a-c: eine dreidimensionale Ansicht eines Stents mit einem an Fig. 2 angelehnten Stentdesign sowie vergrößerte Ausschnitte hieraus, und
- Fig. 4a-c: eine dreidimensionale Ansicht eines Stents mit einem an Fig. 2 angelehnten Stentdesign sowie vergrößerte Ausschnitte hieraus.

Die im Folgenden beschriebenen Stents werden aus einer biodegradierbaren Metalllegierung geformt. Dazu geeignete Metalllegierungen sind in der DE 197 31 021 und DE 199 45 049, beschrieben. Speziell die dort beschriebenen Magnesiumlegierungen mit einer Zusammensetzung von 50-98% Magnesium, 0-40% Lithium, 0-5% Eisen und unter 5% anderer Metalle sowie die dort beschriebenen Eisenlegierungen mit einer Zusammensetzung von 88-99% Eisen, 0,5-7% Chrom und 0,5-3,5% Nickel sowie unter 5% anderer Metalle haben zumindest teilweise eine Bruchdehnung von gleich oder weniger als 30%.

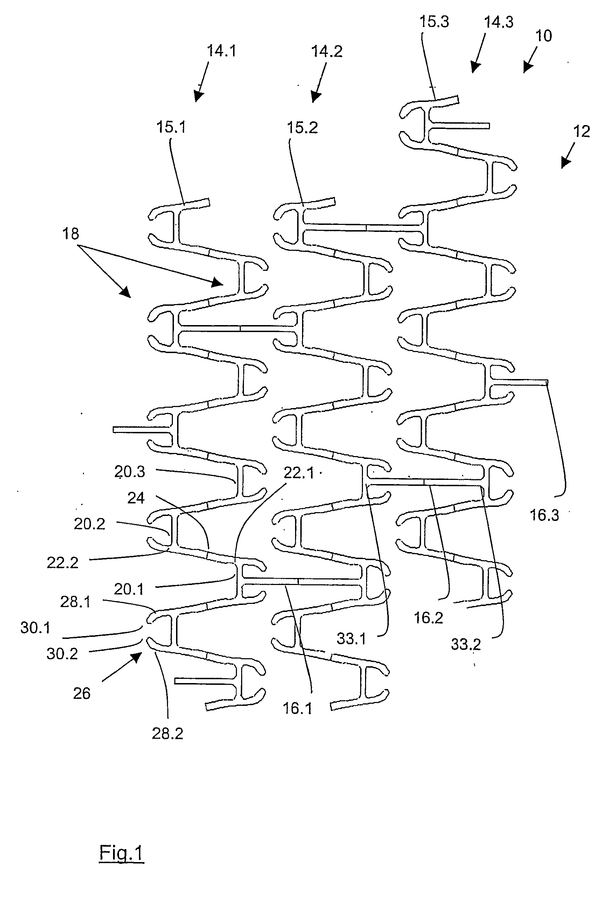

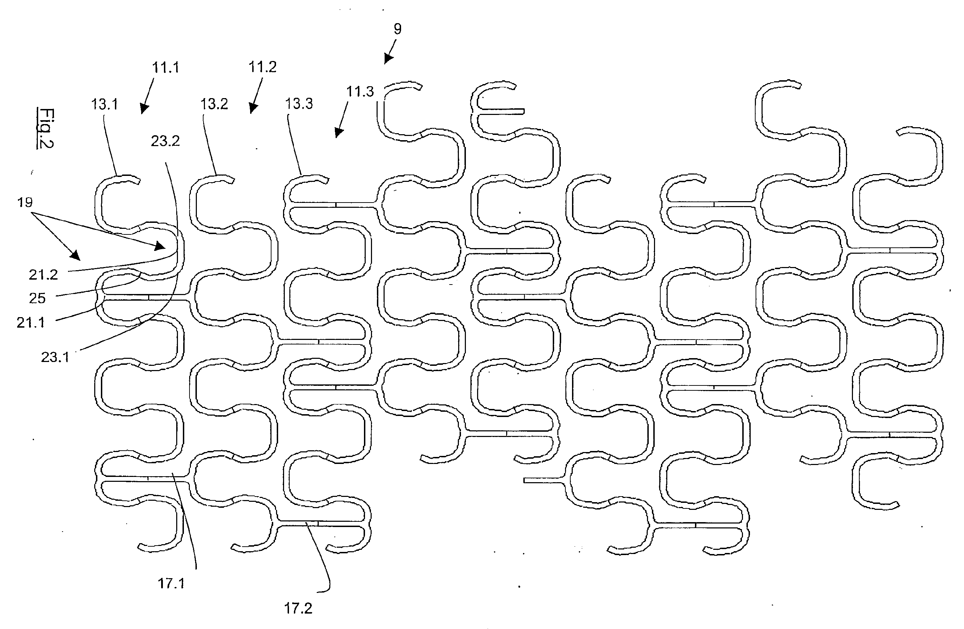

Aufgrund der mechanischen Eigenschaften der genannten Werkstoffe, wie insbesondere ihrer geringen Bruchdehnung, ist ein angepasstes Design erforderlich. Dies wird nachfolgend in den Beispielen der Fig. 1 und 2 näher erläutert:

### Ausführungsbeispiel 1

Die Figur 1 zeigt schematisch einen Teilausschnitt eines Stents 10 und zwar in einer Draufsicht auf eine Abwicklung seiner rohrförmig verlaufenden Umlaufswandung 12. Ein Grundkörper des Stents 10 setzt sich ganz oder in Teilen aus einer Vielzahl von Tragsegmenten 14.1, 14.2, 14.3 zusammen. Die einzelnen Tragsegmente 14.1, 14.2, 14.3 sind über in Längsrichtung des Stents 10 ausgerichtete Querverbinder 16.1, 16.2, 16.3 miteinander verbunden. Die Ausgestaltung der Querverbinder 16.1. 16.2, 16.3 ist hier nur beispielhaft vorgegeben. Zur Erhöhung der Biegeflexibilität können auch Varianten mit S-, V-, W- oder anderen multi-sinus-förmigen Formgebungen genutzt werden.

Die Tragsegmente 14.1, 14.2 und 14.3 der Fig. 1 umfassen eine zickzack- oder wellenförmige Struktur aus einer jeweils um die Längsachse des Stents 10 umlaufenden Strebe 15.1, 15.2, 15.3. Wie ersichtlich weisen die Streben 15.1, 15.2, 15.3 in einem Wendebereich 18 der zickzack- oder wellenförmigen Struktur, d.h. in den Spitzen der einzelnen Mäander, gerade, in Umfangsrichtung des Stents 10 ausgerichtete Biegeabschnitt 20.1, 20.2, 20.3 auf. Die geraden Biegeabschnitte 20.1, 20.2 sind über Verknüpfungspunkte 22.1, 22.2 und einen Schenkelabschnitt 24 miteinander verbunden. Ein Länge eines gerade verlaufenden Biegeabschnitts 20.2 der Strebe 15.1 zu seiner Breite 24 beträgt im konkreten Fall in etwa 1 zu 3,5, wobei die Länge von der geometrischen Mitte der Verknüpfungspunkte 22.1, 22.2 gemessen wird.

Die Querverbinder 16.1, 16.2, 16.3 sind parallel zur Längsrichtung des Stents 10 ausgerichtet und setzen an Ansatzpunkten 33.1, 33.2, etwa in der Mitte jedes sechsten geraden Biegeabschnitts an. Natürlich sind auch andere Ansatzpunkte und Abfolgen denkbar. Wie ersichtlich verlaufen die Streben 15.1, 15.2, 15.3 in Längsrichtung des Stents 10 in Phase, d.h. die Wendebereiche 18 der einzelnen Tragsegmente 14.1, 14.2, 14.3 weisen eine gemeinsame Ausrichtung auf.

Weiterhin zeigt die zickzack- oder wellenförmige Struktur Anschlagelemente 26, die so ausgebildet sind, dass Verformungen der zickzack- oder wellenförmigen Struktur, die in Folge einer Expansion des Stents vom nicht-expandierten in den expandierten Zustand auftreten, im Bereich der geraden Biegeabschnitte 20.1, 20.2, 20.3 vermindert und im Bereich der Schenkelabschnitte 24 verstärkt werden. Im vorliegenden Fall werden die Anschlagelemente 26 von zwei Stegen 28.1, 28.2 gebildet, die mit einem Ende mit der zickzack- oder wellenförmigen Struktur - hier beispielhaft an den Verknüpfungspunkten 22.4 und 22.5 - verbunden sind. Die Stege 28.1, 28.2 sind an ihren freien Enden 30.1, 30.2 derart gekrümmt, dass sie aufeinander gerichtet sind. Während einer Expansion des Stents 10 bewegen sich die freien Enden 30.1, 30.2 aufeinander zu bis sie aneinander liegen. Von diesem Moment an wird eine weitere Verformung der geraden Biegeabschnitte 20.1, 20.2, 20.3 verhindert oder zumindest erschwert. In der Folge findet eine Verlagerung der Verformung in die durch die Schenkelabschnitte 24 gebildeten Verformungszonen des Stents 10 statt.

### Ausführungsbeispiel 2

Die Fig. 2 zeigt schematisch einen Teilausschnitt eines weiteren Stents 9 mit einer Vielzahl von nebeneinander angeordneten Tragsegmenten 11.1, 11.2, 11.3. Die Tragsegmente 11.1, 11.2. 11.3 umfassen eine schlaufenförmige Struktur aus einer um die Längsachse des Stents 9 umlaufenden Strebe 13.1, 13.2, 13.3. Über Querverbinder 17.1, 17.2 sind die einzelnen Tragsegmente 11.1, 11.2, 11.3 in Längserstreckung des Stents 9 miteinander verbunden. Die Darstellung entspricht einer Draufsicht auf eine Abwicklung der aus den einzelnen Tragsegmenten 11.1, 11.2, 11.3 gebildeten Umlaufswandung.

Hinsichtlich der Gestaltungs- und Verknüpfungsmöglichkeiten der Querverbinder 17.1, 17.2 wird auf die im Ausführungsbeispiel 1 genannten Formgebungen verwiesen.

Die Streben 13.1, 13.2, 13.3 zeigen im Wendebereich 19 der schlaufenförmigen Struktur, d. h. an den Spitzen der einzelnen Schlaufen, gerade, in Umfangsrichtung ausgerichtete Biegeabschnitte 21.1, 21.2. Die geraden Biegeabschnitte 21.1, 21.2 sind über zwei Verknüpfungspunkte 23.1, 23.2 und einen leicht doppel-s-förmig gestalteten Schenkelabschnitt 25 miteinander verbunden. Der Schenkelabschnitt 25 dieser Schlaufen ist im Verhältnis zu den geraden Abschnitten 21.1, 21.2 so kurz gehalten, dass er sich bei der Expansion der Stents 9 in Umfangsrichtung nahezu aufrichtet. Ein Verhältnis der Länge zur Breite des geraden Biegeabschnitt 21.2 beträgt im konkreten Fall etwa 1:4, wobei die Länge zwischen den geometrischen Mittelpunkten der Verknüpfungspunkte 23.1, 23.2 gemessen wird.

### Ausführungsbeispiel 3

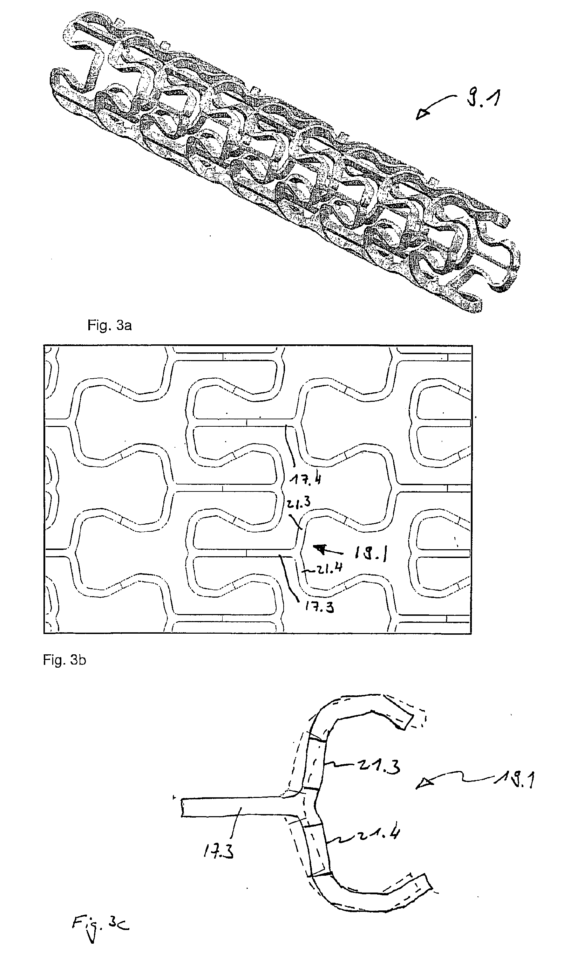

Die Figuren 3a bis 3c zeigen einen Stent 9.1 in dreidimensionaler Ansicht und in vergrößerten Ausschnitten mit einem im Vergleich zum Ausführungsbeispiel 2 nahezu identischen Design. Die einzige Abweichung liegt darin, dass nunmehr in jedem Wendebereich 19.1 Querverbinder 17.3, 17.4 ansetzen. Fig. 3a dient anhand seiner dreidimensionalen Darstellung lediglich der Veranschaulichung des Stentdesigns im nicht-expandierten Zustand. Wie aus dem vergrößerten Ausschnitt der Fig.3b erkennbar, sind die beiden Biegeabschnitte 21.3, 21.4 im Wendebereich 19.1 nicht exakt in Umlaufrichtung des Stents 9.1 ausgerichtet, sondern zeigen eine geringe Abweichung von ca. 8°. Fig. 3c greift den Wendebereich 19.1 mit den beiden Biegeabschnitte 21.3, 21.4 noch einmal vergrößert heraus und deutet zudem anhand der gestrichelten Umrisslinie eine Verformung dieses Bereichs bei Expansion des Stent 9.1 an.

### Ausführungsbeispiel 4

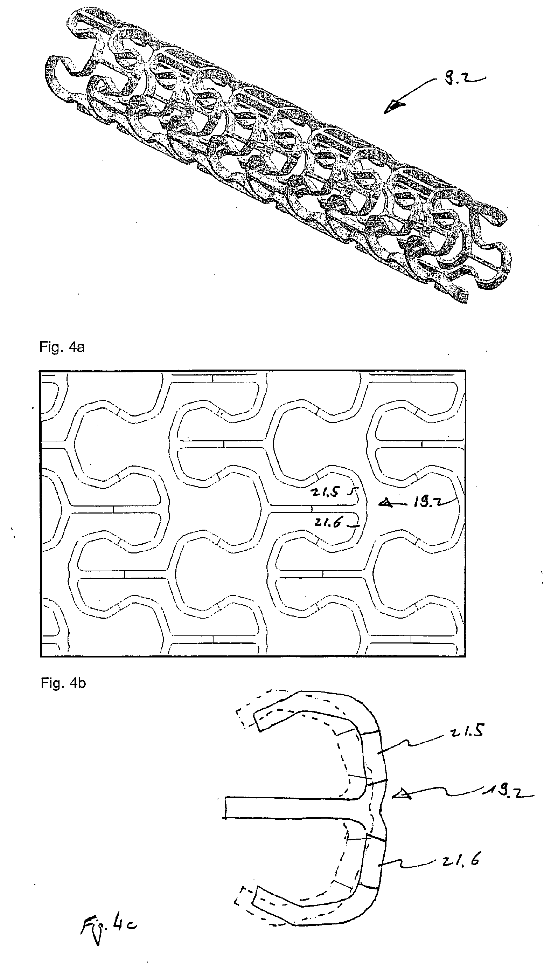

Die Figuren 4a bis 4c zeigen einen Stent 9.2 mit einem im Vergleich zum Ausführungsbeispiel 3 sehr ähnlichen Design. Die Abweichungen liegt lediglich darin, dass zum einen die beiden Biegeabschnitte 21.5, 21.6 im Wendebereich 19.2 gegenüber Ausführungsbeispiel 3 verkürzt sind. Zum anderen weichen die beiden Biegeabschnitte 21.5, 21.6 im Wendebereich 19.2 etwas stärker von der exakten Umlaufrichtung des Stents 9.2 ab, nämlich um ca. 10° (siehe dazu Fig. 4b und 4c). Fig. 4a dient der Veranschaulichung des Stentdesigns im nicht-expandierten Zustand anhand einer dreidimensionalen Darstellung. Fig. 4c greift den Wendebereich 19.2 mit den beiden Biegeabschnitte 21.5, 21.6 noch einmal vergrößert heraus und deutet zudem anhand der gestrichelten Umrisslinie eine Verformung dieses Bereichs bei Expansion des Stent 9.2 an.

Durch die geschilderten Maßnahmen gemäß der Ausführungsbeispiele 1 bis 4 lässt sich erreichen, dass die geraden Biegeabschnitte 20.1-20.3, 21.1-21.6 bei der Expansion der Stents 9, 9.1, 9.2, 10 hauptsächlich durch ein Biegemoment beansprucht werden. Aufgrund des konstanten Hebelarms bzw. des konstanten Moments über die genannten geraden Biegeabschnitte 20.1-20.3, 21.1-21.6 verteilt sich die Dehnung gleichmäßig und lokale Spannungs- und Dehnungskonzentrationen werden weitestgehend vermieden, so dass der geringen Bruchdehnung des Materials Rechnung getragen wird.

## Patentansprüche

1. Stent (9, 9.1, 9.2, 10) aus einer biodegradierbaren Metalllegierung mit einer Bruchdehnung von 30% oder weniger und mit einem rohrförmigen Grundkörper, der ganz oder in Teilen aus Tragsegmenten (11.1-11.3, 14.1-14.3) besteht, die in Längsrichtung des Stents (9, 9.1, 9.2, 10) über Querverbinder (16.1-16.3, 17.1-17.4) miteinander verbunden sind, und die Tragsegmente (11.1-11.3, 14.1-14.3) eine zickzack - oder wellenförmige Struktur aus einer um eine Längsachse des Stents (9, 9.1, 9.2, 10) umlaufenden Strebe (13.1-13.3, 15.1-15.3) umfassen, wobei die Strebe (13.1-13.3, 15.1-15.3) in einem Wendebereich (18, 19, 19.1, 19.2) der zickzack- oder wellenförmigen Struktur einen geraden, in Umfangsrichtung ausgerichteten Biegeabschnitt (20.1-20.3, 21.1-21.6) aufweist.

2. Stent nach Anspruch 1, **dadurch gekennzeichnet, dass** im Wendebereich (18) der zickzack- oder wellenförmigen Struktur Anschlagelemente (26) angeordnet sind, die so ausgebildet sind, dass Verformungen der zickzack- oder wellenförmigen Struktur, die infolge einer Expansion des Stents (10) von einem nicht-expandierten in einen expandierten Zustand auftreten, im Bereich der geraden Biegeabschnitte (20.1-20.3) vermindert und im Bereich der Schenkelabschnitte (24) verstärkt sind.

3. Stent nach Anspruch 2, **dadurch gekennzeichnet, dass** die Anschlagelemente (26) jeweils aus zwei Stegen (28.1, 28.2) bestehen, die mit einem Ende an der zickzack - oder wellenförmigen Struktur fixiert sind und deren freien Enden (30.1, 30.2) aufeinander gerichtet und so angeordnet sind, dass sie sich während der Expansion des Stents (10) aufeinander zu bewegen bis sie aneinander liegen.

4. Stent nach Anspruch 3, **dadurch gekennzeichnet, dass** die Stege (28.1, 28.2) der Anschlagelemente (26) an einem Verknüpfungspunkt (22.1, 22.2, 22.3, 22.4, 22.5) zwischen einem Schenkelabschnitt (24) und dem geraden Biegeabschnitt (20.1-20.3) angreifen.

5. Stent nach Anspruch 1, **dadurch gekennzeichnet, dass** ein Verhältnis einer Länge zu einer Breite des gerade verlaufenden Biegeabschnitts (20.1-20.3, 21.1-21.6) der Strebe (13.1-13.3, 15.1-15.3) 1:2 bis 1:6 beträgt.

6. Stent nach Anspruch 5, **dadurch gekennzeichnet, dass** das Verhältnis 1:2 bis 1:3 beträgt.

7. Stent nach Anspruch 1, **dadurch gekennzeichnet, dass** das Material eine Bruchdehnung von 5 - 30% besitzt.

8. Stent nach Anspruch 7, **dadurch gekennzeichnet, dass** das Material eine Bruchdehnung von 10-30% besitzt.

9. Stent nach Anspruch 8, **dadurch gekennzeichnet, dass** das Material eine Bruchdehnung von 10 - 25% besitzt.

10. Stent nach Anspruch 9, **dadurch gekennzeichnet, dass** das Material eine Bruchdehnung von 10 - 20% besitzt.

11. Stent nach Anspruch 10, **dadurch gekennzeichnet, dass** das Material eine Bruchdehnung von 10-15% besitzt.

12. Stent nach Anspruch 1, **dadurch gekennzeichnet, dass** die biodegradierbare Metalllegierung eine Magnesium-, Eisen-, oder Wolframlegierung ist.

13. Stent nach Anspruch 1, **dadurch gekennzeichnet, dass** die Tragsegmente (11.1-11.3, 14.1-14.3) in Längsrichtung des Stents (9, 9.1, 9.2, 10) derart angeordnet sind, dass die zickzack- oder wellenförmigen Strukturen wenigstens zwei benachbarter Tragsegmente (11.1-11.3, 14.1-14.3) in Phase verlaufen.

14. Stent nach Anspruch 1, **dadurch gekennzeichnet, dass** die Querverbinder (16.1-16.3, 17.1-17.4) parallel zur Längsrichtung des Stents (9, 9.1, 9.2, 10) ausgerichtet sind.

15. Stent nach Anspruch 1 oder 14, **dadurch gekennzeichnet, dass** ein Ansatzpunkt (33.1, 33.2) der Querverbinder (16.1-16.3, 17.1-17.4) im Bereich der geraden Biegeabschnitte (20.1-20.3, 21.1-21.6) liegt.

16. Stent nach Anspruch 15, **dadurch gekennzeichnet, dass** der Ansatzpunkt (33.1, 33.2) in der Mitte der geraden Biegeabschnitte (20.1-20.3, 21.1-21.6) liegt.

17. Stent nach Anspruch 1, **dadurch gekennzeichnet, dass** eine Abweichung der Ausrichtung der geraden Biegeabschnitte (20.1-20.3, 21.1-21.6) in Umfangrichtung des Stents höchstens 25°, insbesondere höchstens 20°, besonders bevorzugt 10° beträgt.

## Claims

1. A stent (9, 9.1, 9.2, 10), made of a biodegradable metal alloy with an elongation at rupture of 30 % or less and with a tubular main body, which consists either completely or in part of supporting segments (11.1-11.3, 14.1-14.3), which are interconnected in the longitudinal direction of the stent (9, 9.1, 9.2, 10) by transverse connectors (16.1-16.3, 17.1-17.4), and the supporting segments (11.1-11.3, 14.1-14.3) have a zigzagged or waved structure formed from a brace (13.1-13.2, 15.1-15.3) extending circumferentially about a longitudinal axis of the stent (9, 9.1, 9.2, 10), wherein, in a turning region (18, 19, 19.1, 19.2) of the zigzagged or waved structure, the brace (13.1-13.3, 15.1-15.3) has a straight bending portion (20.1-20.3, 21.1-21.6) aligned in the circumferential direction.

2. The stent according to claim 1, **characterised in that** stop elements (26) are arranged in the turning region (18) of the zigzagged or waved structure and are designed such that deformations of the zigzagged or waved structure, which occur as a result of expansion of the stent (10) from a non-expanded state into an expanded state, are reduced in the region of the straight bending portions (20.1-20.3) and are intensified in the region of the branch portions (24).

3. The stent according to claim 2, **characterised in that** the stop elements (26) each consist of two webs (28.1, 28.2), which are fixed at one end to the zigzagged or waved structure and of which the free ends (30.1, 30.2) are directed toward one another and are arranged such that they move toward one another during the expansion of the stent (10) until they rest against one another.

4. The stent according to claim 3, **characterised in that** the webs (28.1, 28.2) of the stop elements (26) engage a linking point (22.1, 22.2, 22.3, 22.4, 22.5) between a branch portion (24) and the straight bending portion (20.1-20.3).

5. The stent according to claim 1, **characterised in that** a ratio of length to width of the bending portion (20.1-20.3, 21.1-21.6) of the brace (13.1-13.3, 15.1-15.3) extending in a straight line is 1:2 to 1:6.

6. The stent according to claim 5, **characterised in that** the ratio is 1:2 to 1:3.

7. The stent according to claim 1, **characterised in that** the material has an elongation at rupture of 5-30 %.

8. The stent according to claim 7, **characterised in that** the material has an elongation at rupture of 10-30 %.

9. The stent according to claim 8, **characterised in that** the material has an elongation at rupture of 10-25 %.

10. The stent according to claim 9, **characterised in that** the material has an elongation at rupture of 10-20%.

11. The stent according to claim 10, **characterised in that** the material has an elongation at rupture of 10-15 %.

12. The stent according to claim 1, **characterised in that** the biodegradable metal alloy is a magnesium alloy, iron alloy or tungsten alloy.

13. The stent according to claim 1, **characterised in that** the supporting segments (11.1-11.3, 14.1-14.3) are arranged in the longitudinal direction of the stent (9, 9.1, 9.2, 10) in such a way that the zigzagged or waved structures of at least two adjacent supporting segments (11.1-11.3, 14.1-14.3) are in phase.

14. The stent according to claim 1, **characterised in that** the transverse connectors (16.1-16.3, 17.1-17.4) are aligned parallel to the longitudinal direction of the stent (9,9.1,9.2,10).

15. The stent according to claim 1 or 14, **characterised in that** a start point (33.1, 33.2) of the transverse connectors (16.1-16.3, 17.1-17.4) is situated in the region of the straight bending portions (20.1-20.3, 21.1-21.6).

16. The stent according to claim 15, **characterised in that** the start point (33.1, 33.2) is situated in the middle of the straight bending portions (20.1-20.3, 21.1-21.6).

17. The stent according to claim 1, **characterised in that** a deviation in the alignment of the straight bending portions (20.1-20.3, 21.1-21.6) in the circumferential direction of the stent is 25° at most, in particular 20° at most, and more preferably 10° at most.

## Revendications

1. Endoprothèse (9, 9.1, 9.2, 10), constituée d'un alliage métallique biodégradable présentant un allongement à la rupture de 30 % ou moins et comportant un corps principal tubulaire, qui est composé soit entièrement, soit en partie, de segments porteurs (11.1-11.3, 14.1-14.3), qui sont reliés entre eux dans la direction longitudinale de l'endoprothèse (9, 9.1, 9.2, 10) par des connecteurs transversaux (16.1-16.3, 17.1-17.4), et les segments porteurs (11.1-11.3, 14.1-14.3) ont une structure en zigzag ou ondulée formée d'une contrefiche (13.1-13.2, 15.1-15.3) s'étendant de manière circonférentielle autour d'un axe longitudinal de l'endoprothèse (9, 9.1, 9.2, 10), la contrefiche (13.1-13.3, 15.1-15.3) ayant, dans une région de courbure (18, 19, 19.1, 19.2) de la structure en zigzag ou ondulée, une partie droite de flexion (20.1-20.3, 21.1-21.6) alignée dans la direction circonférentielle.

2. Endoprothèse selon la revendication 1, **caractérisée par le fait que** des éléments d'arrêt (26) sont agencés dans la région de courbure (18) de la structure en zigzag ou ondulée et sont conçus de telle sorte que des déformations de la structure en zigzag ou ondulée, qui surviennent suite à l'extension de l'endoprothèse (10) d'un état non étendu dans un état étendu, sont réduites dans la région des parties droites de flexion (20.1-20.3) et sont intensifiées dans la région des parties de branche (24).

3. Endoprothèse selon la revendication 2, **caractérisée par le fait que** les éléments d'arrêt (26) sont composés chacun de deux bandes (28.1, 28.2), qui sont fixées au niveau d'une extrémité à la structure en zigzag ou ondulée et dont les extrémités libres (30.1, 30.2) sont dirigées l'une vers l'autre et sont agencées de telle sorte qu'elles se déplacent l'une vers l'autre durant l'extension de l'endoprothèse (10) jusqu'à ce qu'elles s'appuient l'une contre l'autre.

4. Endoprothèse selon la revendication 3, **caractérisée par le fait que** les bandes (28.1, 28.2) des éléments d'arrêt (26) sont en prise avec un point de liaison (22.1, 22.2, 22.3, 22.4, 22.5) entre une partie de branche (24) et la partie droite de flexion (20.1-20.3).

5. Endoprothèse selon la revendication 1, **caractérisée par le fait qu'**un rapport de la longueur à la largeur de la partie de flexion (20.1-20.3, 21.1-21.6) de la contrefiche (13.1-13.3, 15.1-15.3) s'étendant en une ligne droite est de 1:2 à 1:6.

6. Endoprothèse selon la revendication 5, **caractérisée par le fait que** le rapport est de 1:2 à 1:3.

7. Endoprothèse selon la revendication 1, **caractérisée par le fait que** le matériau a un allongement à la rupture de 5-30 %.

8. Endoprothèse selon la revendication 7, **caractérisée par le fait que** le matériau a un allongement à la rupture de 10-30 %.

9. Endoprothèse selon la revendication 8, **caractérisée par le fait que** le matériau a un allongement à la rupture de 10-25 %.

10. Endoprothèse selon la revendication 9, **caractérisée par le fait que** le matériau a un allongement à la rupture de 10-20 %.

11. Endoprothèse selon la revendication 10, **caractérisée par le fait que** le matériau a un allongement à la rupture de 10-15 %.

12. Endoprothèse selon la revendication 1, **caractérisée par le fait que** l'alliage métallique biodégradable est un alliage de magnésium, un alliage de fer ou un alliage de tungstène.

13. Endoprothèse selon la revendication 1, **caractérisée par le fait que** les segments porteurs (11.1-11.3, 14.1-14.3) sont agencés dans la direction longitudinale de l'endoprothèse (9, 9.1, 9.2, 10) d'une manière telle que les structures en zigzag ou ondulée d'au moins deux segments porteurs adjacents (11.1-11.3, 14.1-14.3) sont en phase.

14. Endoprothèse selon la revendication 1, **caractérisée par le fait que** les connecteurs transversaux (16.1-16.3, 17.1-17.4) sont alignés parallèlement à la direction longitudinale de l'endoprothèse (9, 9.1, 9.2, 10).

15. Endoprothèse selon la revendication 1 ou 14, **caractérisée par le fait qu'**un point de départ (33.1, 33.2) des connecteurs transversaux (16.1-16.3, 17.1-17.4) est situé dans la région des parties droites de flexion (20.1-20.3, 21.1-21.6).

16. Endoprothèse selon la revendication 15, **caractérisée par le fait que** le point de départ (33.1, 33.2) est situé au milieu des parties droites de flexion (20.1-20.3, 21.1-21.6).

17. Endoprothèse selon la revendication 1, **caractérisée par le fait qu'**une déviation de l'alignement des parties droites de flexion (20.1-20.3, 21.1-21.6) dans la direction circonférentielle de l'endoprothése est d'au plus 25°, en particulier d'au plus 20°, et de manière davantage préférée d'au plus 10°.
